# EUROPEAN PATENT APPLICATION

(11) **EP 4 810 073 A2**
(43) Date of publication of application: **23.09.2026**
(21) Application number: 26182734.9
(22) Date of filing: 27.04.2022
(51) Int. Cl.: A61M 1/00

(54) **SYSTEMS FOR MONITORING NEGATIVE PRESSURE DEVICES**

(30) Priority: 05.05.2021 US 202163184360 P
(62) Document number(s) of the earlier application(s) in accordance with Art. 76 EPC: 22170158.4 / 4 085 877
(71) Applicant: SafeHeal SAS, 75002 Paris (FR)
(72) Inventor: DEPIERRO, Scott, Madison, CT 06443 (US)
(74) Representative: Vossius & Partner Patentanwälte Rechtsanwälte mbB

(57) **Abstract**

The present disclosure provides systems and devices for monitoring the pressure within a negative pressure system, such as a suction cannister. In one aspect, the system includes an anchor configured for positioning within an intestine of the patient at the anastomosis site and a source of negative pressure, such as a suction cannister, fluidly coupled to the anchor. A portable alarm device is coupled to the suction cannister and includes a trigger for detecting the pressure therein. A signal device emits a signal when this pressure is above a threshold level to ensure that the anchor remains securely in contact with the walls of the intestines at the anastomosis site. The alarm device includes a handle to allow a patient to grasp and carry the suction cannister, providing critical patient mobility during the recovery period.

## Description

### FIELD

. The present disclosure generally relates to systems and devices for monitoring negative pressure devices for use with patients and more particularly to portable alarm devices for continuously monitoring the pressure of an internal bypass device to protect against leakage in an anastomosis site following a bowel resection procedure.

### BACKGROUND

. In some instances, a segment of a patient's bowels must be removed to treat certain blockages and diseases, such as colorectal cancer, diverticulitis, severe bleeding, obstructions, and the like. In these bowel resection procedures, a physician dissects and removes a diseased or obstructed portion of the large intestine and then reconnects the healthy ends of the intestine with tiny staples or sutures, often referred to as a bowel anastomosis. The most serious complication occurring with bowel resection procedures is anastomotic leakage, which can occur when the healing process is delayed or incomplete. Anastomotic leakage allows feces and other waste matter to leak into the abdominal cavity, which can lead to a dangerous infection or sepsis. To prevent this from occurring, the anastomosis site must be protected immediately after surgery.

. Traditionally, to prevent anastomotic leakage, surgeons create an external bypass of the anastomosis site, referred to as a diverting ostomy. In this procedure, the surgeon creates a temporary opening or stoma, (e.g., an artificial anus) to divert the intestines to this opening. The surgeon then attaches an ostomy bag to the opening in the abdomen to collect the patient's feces and other waste matter during the healing period. Unfortunately, ostomies are debilitating for patients, prone to complications and costly and inefficient in terms of patient management. In addition, ostomies require a second surgery to reverse.

. To overcome the limitations of diverting ostomy procedures, internal temporary bypass devices have been created. These bypass devices typically include a flexible sheath that is introduced into the colon and placed against the internal walls of the colon at the anastomosis site. The bypass device eliminates any contact between the tissue and feces or other waste matter traveling through the intestines, thereby ensuring the cleanliness of the anastomosis. After healing is complete, the sheath can be removed from the patient through a routine endoscopic procedure without requiring a second surgery.

. One of the challenges with internal bypass devices is ensuring that they remain in secure contact with the bowel tissue around the anastomosis to prevent migration of the sheath or any leakage of bowel contents around the sheath and into the patient's abdomen. To overcome this challenge, bypass devices may include a negative suction or vacuum pressure system that provides a continuous negative pressure to the external walls of the sheath such that the sheath remains in close contact with the internal walls of the bowl around the anastomosis site. This negative suction pressure is typically supplied to the peripheral walls of the sheath with one or more fluid lines or catheters. The fluid lines are coupled to an external source of negative pressure, such as a suction cannister or the like.

. While these new bypass devices have been extremely effective, they suffer from certain drawbacks. For example, continuous suction pressure must be applied to the bypass sheath throughout the entire healing period. If any part of the pressure system suffers a loss in negative pressure, the outer walls of the sheath may lose contact with the intestines and allow migration of the sheath and/or leakage of feces or waste matter through the anastomosis. Accordingly, the pressure of the suction system must be constantly monitored. In the event of a loss of negative pressure, a health care practitioner must be immediately alerted so that the system can be replaced before the sheath ceases to function as intended.

. Another drawback with existing negative pressure systems designed for use with internal bypass devices is that the suction canisters are typically supplied with negative pressure by a separate vacuum device. Therefore, to provide patient mobility during the healing period in the hospital, the storage canisters must either be disconnected from the vacuum device altogether or the patient must move around with the entire system. This not only subjects the patient to traveling with more devices but also with extra tubing, which must be clipped and secured to prevent falls. These troublesome systems are inconvenient to the patient, or to the therapists who assist them in movement.

. To further enhance patient mobility, suction canisters have been designed that can be previously supplied with negative pressure prior to use by the patient. Since these suction cannisters already have a negative or vacuum pressure, they do not require attachment to a separate tube for connection to the vacuum source. Although this provides some improvement in patient mobility, a suction cannister alone is typically difficult for the patient to easily grip and carry.

. What is needed, therefore, are improved systems and devices for protecting an anastomosis site after bowl resection surgery. It would be particularly desirable to provide devices that produce a clear alert signal to the health care professional when there is loss in pressure in the negative pressure system that secures the internal bypass sheath to the anastomosis site. It would be further desirable to provide suction devices that are portable to allow the patient to be mobile during the recovery period to enhance GI motility and improve the patient's overall quality of life during this period.

### SUMMARY

. The following presents a simplified summary of the claimed subject matter in order to provide a basic understanding of some aspects of the claimed subject matter. This summary is not an extensive overview of the claimed subject matter. It is intended to neither identify key or critical elements of the claimed subject matter nor delineate the scope of the claimed subject matter. Its sole purpose is to present some concepts of the claimed subject matter in a simplified form as a prelude to the more detailed description that is presented later.

. The present disclosure provides systems and devices for monitoring negative pressure devices for use with patients. In some aspects, the systems create an internal bypass within a target area of the GI tract of a patient to allow feces and other waste matter to pass through the target area without contacting the walls of the GI tract at the target area. The target area may be, for example, an anastomosis site created after bowel resection surgery to treat certain blockages and diseases, such as colorectal cancer, diverticulitis, severe bleeding, obstructions, and the like.

. In one aspect of the present disclosure, an internal bypass system for an anastomosis site in a GI tract of a patient comprises an anchor configured for positioning within an intestine of the patient at the anastomosis site and a source of negative pressure, such as a suction cannister, fluidly coupled to the anchor. The system further includes an alarm device having a trigger for detecting a pressure in the suction cannister and a signal device configured to emit a signal based on the pressure to ensure that the anchor remains securely in contact with the walls of the intestines at the anastomosis site.

. The systems and devices of the present disclosure respond to the pressure from within the negative pressure system to produce a clear alert signal if any part of the pressure system suffers a loss in suction pressure. This allows a healthcare practitioner to, for example, either diagnose the issue with the negative pressure system or replace one or more components of the system to ensure that the anchor remains in place throughout the healing period, thereby preventing the leakage of feces or waste matter through the anastomosis.

. In certain embodiments, the anchor comprises a flexible sheath having outer walls and an internal channel for allowing passage of matter through the sheath. The anchor may further comprise a stent coupled to the sheath and configured to provide an outward radial force to the sheath. The negative pressure system comprises one or more tubes fluidly coupled to an outer wall of the sheath and at least one suction canister fluidly coupled to the tubes. The suction canister may be coupled to a source of negative or vacuum pressure, or the cannister itself may be the source of negative pressure. The combination of the stent and the negative pressure system secures the flexible sheath against the colonic wall to prevent migration of the sheath and/or leakage around the sheath.

. The alarm device preferably comprises a main body configured for removable attachment to the suction cannister. The main body is configured to allow a patient to grasp the device and carry the suction cannister, thereby providing critical patient mobility during the recovery period.

. In certain embodiments, the main body of the portable alarm device comprises a coupling device for removably attaching the main body to the suction cannister. In some exemplary embodiments, the coupling device comprises a fastener sized to pass through an opening of the suction cannister and an opening of the main body. In some non-limiting aspects, the opening of the suction cannister is a hole located on a projection of the suction cannister, such as a tab or the like.

. In certain embodiments, the trigger may include a movable element disposed in contact with a surface of the suction cannister and configured to move from a first position to a second position when the pressure within the suction cannister reaches the threshold level. In these embodiments, the system further comprises a tactile sensor positioned such that the movable element contacts the tactile sensor when the pressure reaches the threshold level. In an exemplary embodiment, the tactile sensor is coupled to the signal device and configured to activate the signal device when the expandable element contacts the tactile sensor.

. In a preferred embodiment, the movable element is an expandable element that expands in response to the pressure reaching the threshold level. The main body and the suction cannister are positioned by the coupling device such that the surface of the tactile sensor is contactable by the surface of the expandable element in its expanded configuration. In this embodiment, the coupling device serves multiples purposes: to secure the alarm device to the suction cannister for portability and to position the tactile sensor such that the expandable element contacts the tactile sensor upon expansion. Thus, the unique and simple design of the coupling device not only allows for reproducible connection, but is also lightweight and does not burden the patient. Another advantage of the coupling device of some embodiments of the present disclosure, is that it is configured for connecting the main body to suction cannisters that are already widely used in the medical setting, such as the Jet-Vac canister produced by PFM Medical, Inc.

. In certain embodiments, the main body comprises an elongate body having opposing ends configured for removable attachment to first and second suction cannisters. In these embodiments the device comprises first and second sensors. The first sensor is configured to detect pressure within the first suction cannister and the second sensor is configured to detect pressure within the second suction cannister.

. In an exemplary embodiment, the elongate body comprises a handle which enables the patient to easily carry both suction cannisters. In some embodiments, the elongate body is rigid and has a length in the range of about 6 to 18 inches. This provides sufficient space for a handgrip by a patient and for connecting two separate suction cannisters to the alarm device.

. In certain embodiments, the signal device comprises a power supply and suitable electronics coupled to the tactile sensor to produce an alarm or alert signal indicating that the internal pressure of the suction cannister is above the threshold level. In other embodiments, the power supply and electronics may be separate from the alarm device and coupled to the tactile sensor through a wireless connection, such as Bluetooth or the like. The alarm or alert signal may be any signal that alerts the patient and/or the health care professional that the container(s) are full and must be replaced. For example, the signal may comprise an audible alert, a visual alert or it may be transmitted by direct connection or wirelessly to a remote location, such as the nurse station or other suitable location.

. In another aspect of the invention, a portable alarm device is provided for use with an internal bypass system for an anastomosis site. The alarm device comprises a main body configured for removable attachment to a suction cannister and at least one sensor coupled to the main body and configured to detect a pressure from an interior of the suction cannister. The alarm device further includes a signal device coupled to the sensor and configured to emit a signal when pressure within the suction cannister is above a threshold level. One of the advantages of this alarm device is that main body comprises a elongate portion that functions as a handle to allow a patient to grasp the device and carry the suction cannister, thereby providing patient mobility during the recovery period.

. In certain embodiments, the main body of the portable alarm device comprises a coupling device for removably attaching the main body to the suction cannister. In some exemplary embodiments, the coupling device comprises a fastener sized to pass through an opening of the suction cannister and an opening of the main body. In some non-limiting aspects, the opening of the suction cannister is a hole located on a projection of the suction cannister, such as a tab or the like.

. In certain embodiments, the system may include an expandable element disposed in contact with a surface of the suction cannister and configured to expand when the pressure within the suction cannister reaches the threshold level. In these embodiments, the sensor comprises a tactile sensor positioned such that the expandable element contacts the tactile sensor when the pressure reaches the threshold level. In an exemplary embodiment, the tactile sensor is coupled to the signal device and configured to activate the signal device when the expandable element contacts the tactile sensor.

. In a preferred embodiment, the main body and the suction cannister are positioned by the coupling device such that the surface of the tactile sensor is contactable by the surface of the expandable element of the suction cannister in its expanded configuration. In this embodiment, the coupling device serves multiples purposes: to secure the alarm device to the suction cannister for portability and to position the tactile sensor such that the expandable element contacts the tactile sensor upon expansion.

. In another aspect of the invention, a portable alarm device is provided for use with an internal bypass system for an anastomosis site. The alarm device comprises an elongate body with first and second opposing ends. Each opposing end is configured for removable attachment to first and second suction cannisters. The device further comprises a first trigger configured to detect a pressure from an interior of the first suction cannister and a second trigger configured to detect a pressure from an interior of the second suction cannister. The device further comprises a signal device coupled to each trigger and configured to emit a signal when the pressure within the first or second suction cannister is above a threshold level.

. According to an exemplary embodiment, the elongate body comprises a handle that can be easily grasped and carried by the patient. In some embodiments, the elongate body of the device is rigid and has a length in the range of about 6 to 18 inches. An elongate body having a length in this range would allow the device to connect to two canisters at once, one at each end.

. In some embodiments, the alarm device comprises first and second coupling devices for removably attaching the elongate body to the first and second suction cannisters. In an illustrative embodiment, each of the first and second coupling devices comprises a fastener sized to pass through an opening of each of the first and second suction cannisters and an opening of the main body. This unique design of a three-part portable system lends itself to improving patient mobility during the recovery period.

. In certain embodiments, the first and second triggers each comprise an element having a contact surface configured to contact a surface of an expandable element of one of the first and second suction cannisters, the expandable element being configured to expand when the pressure within one of the first and second suction cannisters reaches the threshold level. In other embodiments, the device further comprises first and second tactile sensors positioned such that the expandable element of one of the first and second suction cannisters contacts the element of one of the first and second tactile sensors when the pressure reaches the threshold level.

. In an exemplary embodiment, the first and second tactile sensors are coupled to the signal device and configured to activate the signal device when an expandable element of either one or both of the first and second suction cannisters contacts one of the tactile sensors. The elongate body of the portable device and the first and second suction cannisters may be positioned by the first and second coupling devices such that the surface of the element of one of the first and second tactile sensors is contactable by the surface of the expandable element of one of the first and second suction cannisters in its expanded configuration.

. In exemplary embodiments, the signal device comprises a power supply. In certain embodiments, the signal comprises an audible alert. In other embodiments, the signal comprises a visual alert. In yet other exemplary embodiments, the signal comprises an audible alert. In some embodiments, the signal comprises data and the device further comprises a transmitter for transmitting the data to a remote location. It is understood that the signal may also be a combination of any of these types of alerts.

### BRIEF DESCRIPTION OF THE DRAWINGS

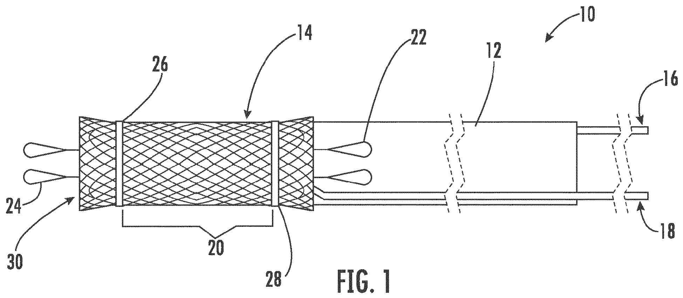

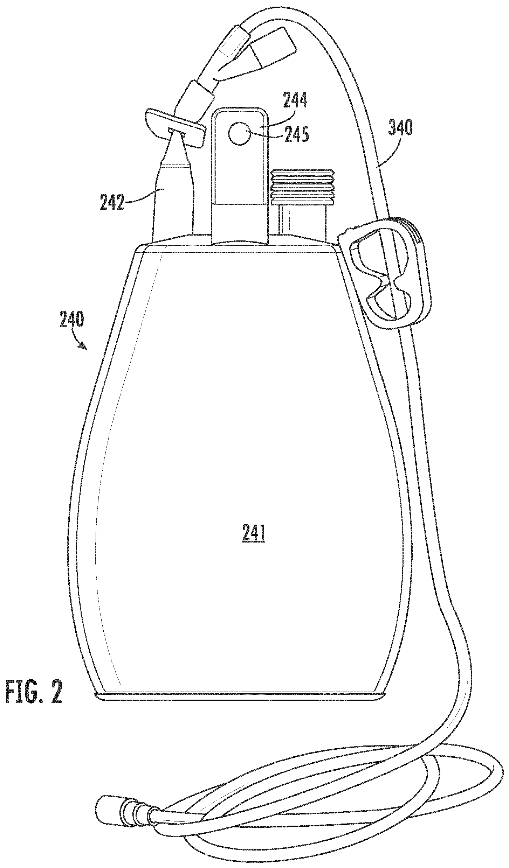

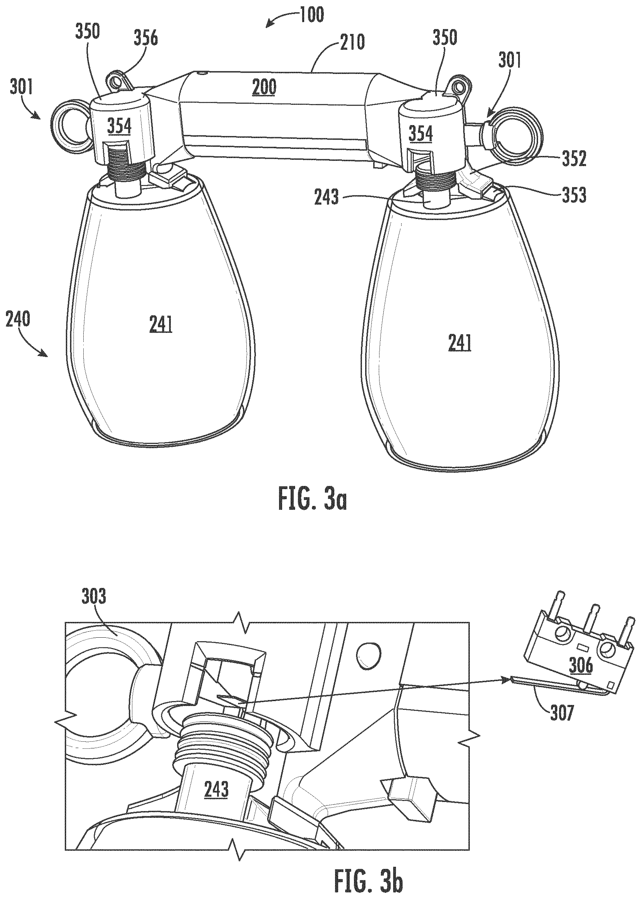

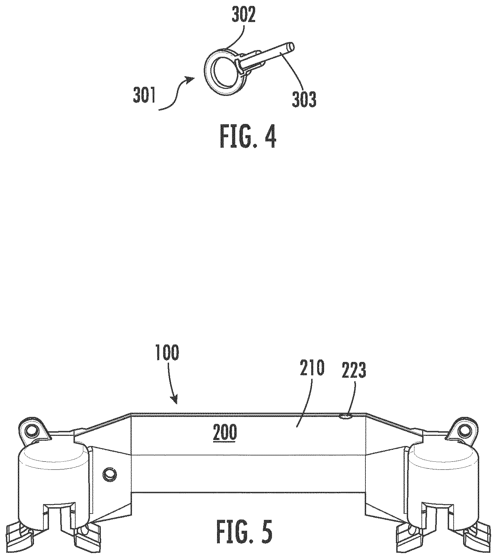

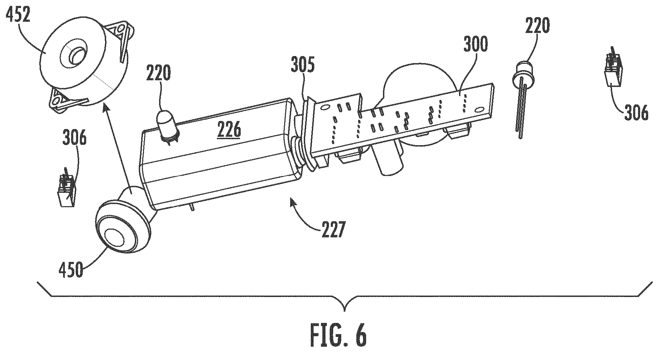

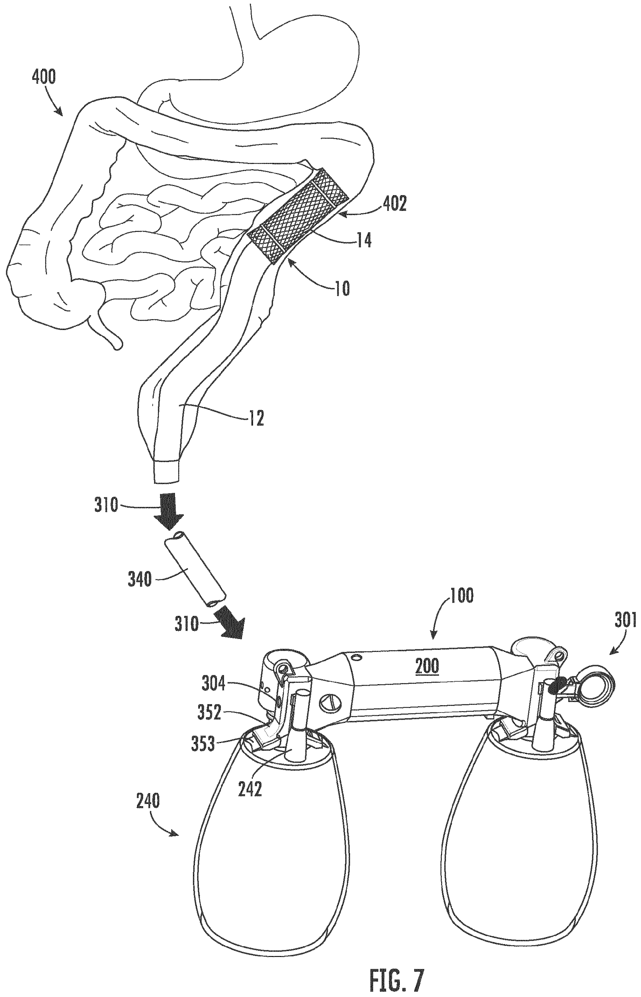

. The accompanying drawings, which are incorporated in and constitute a part of this specification, illustrate several embodiments of the disclosure and together with the description, serve to explain the principles of the disclosure.
. FIG. 1 is a side view of an anchor for an internal bypass system according to the present disclosure;
. FIG. 2 is a perspective view of an exemplary suction cannister for use with an internal bypass system according to the present disclosure;
. FIG. 3A is a perspective view of a portable alarm device of the present disclosure coupled to two representative suction cannisters;
. FIG. 3B is an exploded view of a portion of the portable alarm device of FIG. 3A.
. FIG. 4 is a perspective view a coupling device for removably attaching the alarm device of FIG. 3A to a suction cannister;
. FIG. 5 is a perspective view of a main body of the portable alarm device of FIG. 3A;
. FIG. 6 is an exploded view of a signal device of the portable alarm device of FIG. 3A; and
. FIG. 7 illustrates the internal bypass device of the present invention in use at an anastomosis site within the GI tract of a patient.

### DETAILED DESCRIPTION OF THE PREFERRED EMBODIMENTS

. Particular embodiments of the present disclosure are described hereinbelow with reference to the accompanying drawings; however, it is to be understood that the disclosed embodiments are merely exemplary of the disclosure and that the disclosure may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure. Well-known functions or constructions are not described in detail to avoid obscuring the present disclosure in any unnecessary detail. It should be understood also that the drawings are not drawn to scale and are not intended to represent absolute dimensions or relative size. Instead, the drawings help to illustrate the concepts described herein.

. The present disclosure provides systems and devices for creating an internal bypass within a target area of the GI tract of a patient to allow feces and other waste matter to pass through the target area without contacting the walls of the GI tract at the target area. The target area may be, for example, an anastomosis site in the colon, rectum or anal passage created after bowel resection surgery to treat certain blockages and diseases, such as colorectal cancer, diverticulitis, severe bleeding, obstructions and the like. The bypass device may be secured to the anastomosis site with a negative pressure system, such as one or more suction cannisters coupled to the bypass device via suitable medical tubing, or the like. The present disclosure further provides a portable alarm device that responds to the pressure from within the suction cannister(s) and produces an alert signal to the health care professional when the pressure reaches a threshold level that would no longer be sufficient to secure the bypass device to the anastomosis site.

. The portable alarm device is coupled to one or more suction cannisters and provides a handle for grasping and holding the cannister(s). This design allows the storage canisters to be portable to allow the patient to walk within their recovery room or about the halls of the hospital, as their normal mobility is restored, without disrupting the continuous application of negative or vacuum pressure to the bypass device.

. Referring now to Fig. 1, an anchor 10 for providing an internal bypass at a target site within the GI tract of the patient will now be described. As shown, anchor 10 comprises an elongate flexible sheath 12 having outer walls and an internal channel for allowing passage of feces and other waste matter therethrough. Sheath 12 has a length selected to extend from the target site in the GI tract through the patient's intestines and anus to a location external to the patient (see Fig. 7). Sheath 12 includes a proximal end coupled to a collection vessel (not shown) that is located within, or external to, the patient's body. Sheath 12 may comprise a biocompatible elastomer material, such as silicone or polyurethane.

. Anchor 10 further comprises a stent 14 coupled to sheath 12 and configured to provide an outward radial force against sheath 12 or against the walls of the intestines. Stent 14, in combination with the negative pressure system described below, functions to hold open the target area within the GI tract such that feces and other waste matter will pass through sheath without contacting the walls around the anastomosis site. A more complete description of a suitable sheath and stent for use in bypass device 10 can be found in U.S. Patent No. 9,339,272, the complete disclosure of which is incorporated herein by reference for all purposes.

. Bypass device 10 further comprises one or more suction tubes 16, 18 extending along the outer periphery of sheath 12. Suction tubes 16, 18 have proximal ends that are coupled to a negative or vacuum pressure system for providing a negative pressure around the walls of sheath 12 and/or stent 14. This negative pressure functions to suction the outer walls of sheath 12 and stent 14 within a vacuum chamber area 20 to the inner walls of the intestines. In certain embodiments, bypass device may further include a distal covering ring 30 and one or more sealing rings 26, 28 extending around stent 14 to securely fasten stent 14 to the distal end of sheath 12. In addition, device 10 may include distal and/or proximal retrieval loops 22, 24 for facilitating removal of the device 10 after the anastomosis has fully healed.

. As shown in FIG. 2, the negative pressure system of the present disclosure further comprises one or more suction cannister(s) 240 coupled to suction tubes 16, 18 in FIG 1 via medical tubing 340. Suction cannister 240 provides a source of negative or vacuum pressure to suction tubes 16, 18. In some embodiments, suction cannister 240 is a standard suction cannister made of a lightweight plastic, such as those provided by PFM Medical, Inc. In other embodiments, suction cannister 240 may be another fluid management system known in the medical art. For example, the container may be the Medi-Vac suction canister sold by Cardinal Health, Inc., which is also a rigid plastic container but has a different structure than that of the Jet-Vac canister. In yet other embodiments, suction cannister 240 is a custom-designed one for use with the portable device of this disclosure.

. Suction cannister 240 further comprises a projection 244 with an opening 245 on its top surface. In some embodiments discussed below, the main body of the portable device or accessory components to the portable device may comprise an insert, such as a fastener, which is sized to pass through opening 245. In the embodiment depicted in FIG. 2, the projection 244 of the suction canister is a plastic tab that includes opening 245. It is understood that suction cannister 240 may comprise a different structure altogether than what is shown in FIG. 2. For example, suction cannister 240 may not have projection 244 and the coupling to the portable alarm device 100 may be accomplished by a different mechanism than that of the fastener described below.

. The representative suction cannister 240 further comprises an inflow adaptor 242. Inflow adaptor 242 is capable of further attachment to medical tubing 340. In other embodiments of suction cannister 240, inflow adaptor 242 may be located elsewhere on the container instead of its top surface. For example, the location of inflow adaptor 242 may actually be somewhere near the bottom of the suction cannister. In some embodiments, it may be useful for the inflow adaptor 242 to comprise a control valve to allow for starting or stopping flow.

. FIG. 3a is a perspective view of a portable alarm device 100 according to the present invention. As shown, alarm device 100 comprises a main body 200 removably coupled to two suction cannisters 240. Main body 200 comprises an elongated portion 210, suitable for accommodating a handgrip of the average-sized patient's hands, and two opposing ends 350. Main body 200 or its elongated portion 210 may be about 6-10 inches in length to accommodate the coupling of two canisters, such as two 600mL Jet-Vac canisters. Alternatively, main body 200 or elongated portion 210 may be longer than 10 inches to couple to wider-diameter canisters, or to accommodate a larger-size handgrip. In a preferred embodiment, the total length of main body 200 or elongated portion 210 is no more than 18 inches so that device 100 is lightweight and manageable to move. While these are descriptions of the preferred lengths of main body 200 or elongated portion 210, it should be understood that device 100 may be shorter or longer than the lengths described herein.

. Elongated portion 210 has a generally angular shape, like a rectangular block. The perpendicular cross-section of elongate portion 210, therefore, may be irregularly shaped and include sharp angles. In other embodiments, elongate portion 210 of main body 200 has a generally tubular or curvilinear shape, such as with a curved surface, similar to a standard bicycle handlebar. In these instances, the cross section of elongate portion 210 is generally circular or oval in nature. Main body 200 may alternatively be a combination of these shapes, with angular edges at opposing ends 350 of the device and tubular in the middle portion where the hand surrounds it. Main body 200 may be substantially rigid to be suitable for a robust grip. However, it may be at least partially covered with rigid or non-rigid padding to increase comfort while a patient holds it. The main body 200 may alternatively be covered at least in part by a material having roughness suitable for a non-slip hand grip. The covering could instead be a disposable liner. No matter the material, the surface of the device or the covering should be such that can be disposed of or easily disinfected with standard cleaning supplies used for cleaning medical equipment known in the art.

. Elongate portion 210 of device 100 serves as a handle while opposing ends 350 include the coupling assemblies. In this embodiment, since opposing ends 350 are not constituents of the handle portion of the device, they may have a different shape altogether. For example, opposing ends 350 may include the coupling device and sensors and thus, may be modularly designed and irregularly shaped.

. One embodiment of opposing ends 350 is shown in FIG. 3a. In this embodiment, each opposing end 350 comprises a hollow partial enclosure 354 for receiving and partially enclosing an expandable element 243 of suction cannister 240, which is discussed in further detail below. Opposing ends 350 further include an opening 304 shown more clearly in FIG. 7. Opening 304 is sized for removable insertion of a fastener, such as a pin 303, shown in FIG. 4. In the exemplary embodiment shown in FIG. 3a, each opposing end 350 further includes a hanging tab 356 configured to allow the patient or clinician the option of hanging the device, whether alone or while coupled to suction cannisters 240. This would be useful in the instances when the patient's hands are occupied as they move about. For example, in the earlier stages of their recovery, the patient may be asked by a physical therapist to walk while holding onto a walker with both hands. In another example, the patient may find it more convenient to hang the device bedside to keep surfaces in their immediate vicinity clear for other uses.

. Opposing end 350 may further include a base 352 that engages with the surface of suction cannister 240 and at least partially houses projection 244 of suction cannister 240. In some embodiments, as shown in FIG. 3a and more clearly in FIG. 7, base 352 includes a partially hollow enclosure with protruding feet 353 that make contact with top surface of suction cannister 240. For example, projection 244 may be inserted into the hollow space within base 352 so that its opening 245 would align with corresponding opening 304. In this manner, base 352 and projection 244 become locked in position when pin 303 is removably inserted through both openings. It should be understood that there may be multiple different embodiments of the surface of suction cannister 240 and coupling device 301, and therefore the design of the device, including its opposing ends 350, will be contingent on the structure necessary to achieve a secure connection between the device and container.

. In some embodiments, suction cannister 240 comprises an expandable element 243 on a surface of cannister 240. Expandable element 243 may be in a contracted configuration as exemplified in the image of the right suction cannister 240 of FIG. 3a, or in an expanded configuration as shown on the left suction cannister 240 in FIG. 3a. In other embodiments, expandable element 243 may be an integral piece of suction cannister 240. In other embodiments, expandable element 243 is a separate component suitably coupled to suction cannister 240. Expandable element 243 may be a component of portable alarm device 100. In these embodiments, other components of suction cannister 240 transduce a signal of the pressure reaching a threshold level to expandable element 243, forcing it to expand. Regardless of its location, expandable element 243 is configured to expand in response to the pressure within suction cannister 240.

. In an exemplary embodiment, expandable element 243 is configured to expand when the pressure within the suction cannister 240 reaches a threshold level. For example, if there is a loss of negative or suction pressure, the threshold pressure is reached, which causes expandable element 243 to move into the expanded configuration. In an exemplary embodiment, when expandable element 243 expands into its expanded configuration, its surface makes contact with the surface of a sensor element 307 of a tactile sensor 306 of device 100, as shown in the exploded portion of FIG. 3b.

. The threshold pressure level that will trigger expandable element 243 may vary depending on the suction cannister used with the system. At a minimum, this threshold level will comprise a pressure that is high enough such that the internal bypass device is no longer capable of holding open the target area within the GI tract such that feces and other waste matter will pass through sheath without contacting the walls around the anastomosis site. In certain embodiments, the threshold pressure level will be based on an actual pressure value. A vacuum or suction pressure can generally be created with a pressure level below atmospheric pressure, or below about 760 mm Hg, which will vary based on the altitude of the suction canister, the temperature and other factors. In order to provide sufficient suction pressure to the internal bypass device, the pressure within the suction cannister is preferably at a level well below atmospheric pressure, e.g., about 480 mm Hg, or less. In certain embodiments, the threshold pressure level is a pressure greater than about 400 mm Hg, preferably greater than about 480 mm Hg.

. In other embodiments, the threshold pressure level will be based on a percentage of the pressure level set by the individual suction canister. For example, certain commercial cannisters are set such that the trigger will expand when the pressure within the cannister is about 80% of the predetermined vacuum pressure within the system. In other cases, the pressure level may be set at other percentages, such as 90%, 75%, 50%, etc., depending on the capabilities of the individual suction cannister.

. It will be recognized by those skilled in the art that expandable element 243 may have a different structure than described above. For example, element 243 may be rectangular, circular, square, conical, or domed-shaped. In addition, element 243 may be designed to move from one position to another, or to simply translate or extend in one direction, when the threshold pressure has been reached in cannister 240 (rather than expanding).

. In certain embodiments, sensor 306 is a type of pressure sensor, such as a tactile pressure sensor. In some embodiments, a surface of the tactile pressure sensor is configured to be contacted by the surface of expandable element 243 of FIG. 3b. In certain embodiments, the tactile sensor is further coupled to signal device 227. In one aspect, as shown in FIG. 3b, element 307 of sensor 306 has a surface which is acted upon by the expansion of the expandable element 243. It should be understood that there are many different types of switches, including snap-action switches, such as sensor 306 of FIG. 2b, which may be used to detect the force of expansion of expandable element 243.

. FIG. 4 illustrates an exemplary embodiment of a coupling device 301 for removably attaching device 100 to suction cannister 240. In this embodiment, coupling device 301 allows main body 200 of device 100 to engage with the top surface of suction cannister 240. Coupling device 301 includes a fastener for retaining device 100 to suction cannister 240. In certain embodiments, the fastener is a pin 303 which may further comprise a quick-release push-button along its body. Once pin 303 is inserted into opening 304, its exposed end may be further attached to a pin handle 302. Pin handle 302 facilitates fastening or releasing device 100 to or from suction cannister 240.

. In certain aspects, projection 244 of the suction cannister 240 is a tab which has opening 245. For example, the top surface of each of first and second suction cannisters 240 comprises a protruding tab with an opening 245 that allows for the insertion of a fastener. However, it is understood that the surface of the suction cannister may comprise a different structure. For example, suction cannister 240 may not have projection 244 and the coupling may be accomplished using something other than pin 303 or other type of fastener. For example, other types of connections may removably attach main body 200 to suction cannister 240. In one aspect, a clamp may be placed around the body or neck of suction cannister 240. The clasping end of the clamp may secure suction cannister 240 in place while the other end of the clamp may be attached to or inserted through main body 200 of device 100. In such aspects, the coupling of suction cannister 240 to portable alarm device 100 would be accomplished using something similar to pronged extension clamps that hold chemical flasks in place.

. In a preferred embodiment, device 100 includes a coupling device 301 on each opposing end 350 for attaching device 100 to two separate suction cannisters 240. Main body 200 of device is configured such that the patient may grasp and carry device 100 with two suction canisters about the room or halls of the hospital without disrupting the suction pressure applied to bypass device 10.

. FIG. 5 is a perspective view of main body 200 of alarm device 100. As previously mentioned, a length of about 6 inches of elongated portion 210 or main body 200 would result in a lighter weight system and would accommodate connections to smaller, low-volume canisters. Such systems may perhaps be used for young patients or those with small hands. Alternatively, a rigid main body 200 or elongated portion 210 having a length in the range of about 6 to 10 inches provides an easily maneuverable three-dimensional object with sufficient space for the average-sized patient's grip, giving the patient the ability to balance and control two suction cannisters. Alternatively, a device length above 15 inches would accommodate connecting to two canisters that are very large and may perhaps each have a diameter of greater than 6 inches, for example. It is understood that these device lengths are only examples of device lengths and the device's main body 200 may be longer or shorter than described herein.

. In an exemplary embodiment, an LED illumination window 223 can be seen which allows an LED unit 220 (see Fig. 6) to shine light therethrough a signal that pressure from within the canister has reached a threshold value. In other embodiments, the signal comprises an audible alert. In other embodiments, the signal comprises both an audible and a visual alert. In yet other embodiments, the signal comprises data transmitted to a remote location. The signal may also be a combination of one or more types of alerts. Regardless of the alert type, the signal indicates that there has been a loss of negative pressure within the system, which may cause bypass device 10 to migrate or become less secure to the intestinal walls of the patient.

. FIG. 6 is an exploded view of an embodiment of an exemplary signal device 227 for providing an alert that there has been a loss in suction pressure in the system. In certain embodiments, signal device 227 comprises an electronics assembly which includes a power supply 226, such as a battery, coupled to a circuit board 300 via a suitable connector 305. If device 100 is configured to emit an audible signal, then a buzzer 452 is present, shown in the exploded view as a component behind or internal to a mute button 450 that silences buzzer 452. The surface of mute button 450 is accessible to the patient or user for silencing the alarm. It is understood that other types of silencers and buzzers, including micro-buzzers, may be used in device 100.

. In some embodiments, there may be two LED units 220, a mute button 450, and a buzzer 452. It is understood that components of signal device 227 may be configured in multiple different ways. For example, a power supply 226 may be disposed external to main body 200 of alarm device 100. The battery of power supply 226 may be a small, rechargeable battery and circuit board 300 may be shaped so as to fit power supply 226 and accompanying electronics within another section of the device 100. For example, power supply 226 may be housed within opposing end 350 of FIG. 2a. Device 100 may further have hardware for transmitting and receiving a Wi-Fi signal. It is understood that the components of signal device 227 and other electronics and adaptors may vary, and the disclosure herein is not limited to those embodied by FIG. 6.

. In use, bypass device 10 is introduced endoscopically through the patient's rectum and into the large intestines 400, and then advanced to the anastomosis site 402. Stent 14 is expanded through any suitable manner known in the art and suction tubes 16, 18 are coupled to one or more suction cannisters 240 via medical tubing 340. Suction pressure is then applied to sheath 12 via suction tubes 16, 18 (see FIG. 1) to secure device 10 against the intestinal tissue. Device 10 is now properly placed to provide an internal bypass of intestinal contents such that these contents do not contact the anastomosis site, or leak through the anastomosis. A more complete description of the method of inserting device 10 into the patient is described in U.S. Patent No. 9,339,272, previously incorporated herein by reference.

. Portable alarm device 100 is coupled to each suction cannisters 240 by sliding projection 244 into base 352 such that protruding feet 353 are seated on the surface of the canister. In these embodiments, it is important that opening 245 of projection 244 aligns with open space 304 within the base 352. In this manner, pin 303 may be inserted through both openings and click into place when the quick-release button on its end reaches the housing space within opposing end 350 of device 100.

. In a preferred embodiment, main body 200 and suction cannisters 240 are positioned by coupling device 301 such that element 307 of the tactile sensor is contactable by expandable element 243 of suction cannister 240 once pressure within the canister reaches a threshold value. Coupling device 301 is a unique and simple design that allows reproducibility in the precise configuration of suction cannisters 240 coupled to device 100. The coupling mechanism is also inherently lightweight and thus, does not further burden the patient.

. If the pressure reaches a threshold value within the negative pressure system, element 243 expands, contacting element 307 of sensor 306 and signals the alarm as described above. This immediately alerts the patient or a healthcare practitioner that suction pressure has been lost and the system must be diagnosed or replaced to ensure that bypass device remains securely in place at the anastomosis site.

. The devices in the present disclosure provide an easily graspable and lightweight design to enhance movement of the patient for personal needs or for performing exercises, such as for physical therapy. This allows the patient to be mobile during the recovery period, improving overall quality of life during the entire recovery window.

. For example, in a first aspect, a first embodiment comprises a portable alarm device for use with a negative pressure system. The portable alarm device comprises a main body configured for removable attachment to a suction cannister, the main body including a handle, at least one trigger coupled to the main body and configured to detect a pressure from an interior of the suction cannister and a signal device coupled to the trigger and configured to emit a signal.

. A second embodiment is the first embodiment, wherein the main body comprises a coupling device for removably attaching to the main body to the suction cannister.

. A third embodiment is the second embodiment, wherein the coupling device comprises a fastener sized to pass through an opening of the suction cannister and an opening of the main body.

. A fourth embodiment is any combination of the first three embodiments, wherein the trigger comprises an expandable element coupled to an outer wall of the suction canister, the expandable element being configured to expand when a pressure of the suction canister reaches a threshold level.

. A fifth embodiment is any combination of the first four embodiments, further comprising a tactile sensor having a contact surface configured to contact a surface of the expandable element of the suction canister when the main body is coupled to the suction cannister.

. A sixth embodiment is any combination of the first five embodiments, wherein the tactile sensor is positioned such that the expandable element contacts the tactile sensor when the pressure reaches the threshold level.

. A seventh embodiment is any combination of the first six embodiments. wherein the tactile sensor is coupled to the signal device and configured to activate the signal device when the expandable element contacts the tactile sensor.

. An eighth embodiment is any combination of the first seven embodiments. wherein the main body comprises an elongate body having opposing ends configured for removable attachment to first and second suction cannisters.

. A ninth embodiment is any combination of the first eight embodiments wherein the trigger is a first trigger and wherein the first trigger is configured to detect pressure within the first suction cannister, the device further comprising a second trigger configured to detect pressure within the second suction cannister.

. A tenth embodiment is any combination of the first nine embodiment, wherein the elongate body comprises the handle.

. An eleventh embodiment is any combination of the first ten embodiments, wherein the signal comprises an audible alert.

. A twelvth embodiment is any combination of the first eleven embodiments wherein the signal comprises a visual alert.

. A 13^{th} embodiment is any combination of the first 12 embodiments, wherein the signal comprises data and the device further comprises a transmitter for transmitting the data to a remote location.

. In a second aspect, a first embodiment is a portable device for use for use with a negative pressure system, the device comprising an elongate body with first and second opposing ends, wherein the first and second ends are configured for removable attachment to first and second suction canisters, a first trigger configured to detect a pressure from an interior of the first suction canister and a second trigger configured to detect a pressure from an interior of the second suction canister and a signal device coupled to each trigger and configured to emit a signal

. A second embodiment is any combination of the first embodiment wherein the elongate body comprises a handle.

. A 3rd embodiment is any combination of the first two embodiments wherein the alarm device comprises first and second coupling devices for removably attaching the elongate body to the first and second suction canisters.

. A 4^{th} embodiment is any combination of the first three embodiments wherein each of the first and second coupling devices comprises a fastener sized to pass through an opening of each of the first and second suction canisters and an opening of the main body.

. A fifth embodiment is any combination of the first four embodiments wherein the first and second triggers each comprise an expandable element configured to expand when the pressure within one of the first and second suction canisters reaches the threshold level.

. A 6th embodiment is any combination of the first five embodiments further comprising first and second tactile sensors positioned such that the expandable elements contacts one of the first and second tactile sensors when the pressure reaches the threshold level.

. A 7th embodiment is any combination of the first six embodiment wherein the first and second tactile sensors are coupled to the signal device and configured to activate the signal device when an expandable element of either one or both of the first and second suction cannisters contacts one of the tactile sensors.

. Persons skilled in the art will understand that the devices and methods specifically described herein and illustrated in the accompanying drawings are non-limiting exemplary embodiments. The features illustrated or described in connection with one exemplary embodiment may be combined with the features of other embodiments. Various alternatives and modifications can be devised by those skilled in the art without departing from the disclosure. Accordingly, the present disclosure is intended to embrace all such alternatives, modifications, and variances. As well, one skilled in the art will appreciate further features and advantages of the present disclosure based on the above-described embodiments. Accordingly, the present disclosure is not to be limited by what has been particularly shown and described, except as indicated by the appended claims.
By way of example, embodiments of the invention comprise the following features:
1. An internal bypass system for a target site in a GI tract of a patient, the system comprising:
   an anchor configured for positioning within the GI tract of the patient at the target site;
   a negative pressure system having a source of negative pressure fluidly coupled to the anchor;
   an alarm device coupled to the negative pressure system and having a trigger for detecting a pressure from within the negative pressure system; and
   a signal device coupled to the trigger and configured to emit a signal.
2. The bypass system of item 1, wherein the anchor comprises a flexible sheath having outer walls and an internal channel for allowing passage of matter through the sheath.
3. The bypass system of item 1 or 2, wherein the anchor further comprises a stent
   coupled to
   the sheath and configured to provide an outward radial force to the sheath.
4. The bypass system of item 1, 2 or 3, wherein the negative pressure system
   comprises one or
   more tubes fluidly coupled to an outer wall of the sheath and at least one suction canister fluidly coupled to the one or more tubes.
5. The bypass system in any of items 1 to item 4, wherein the alarm device comprises
   a main body having a
   coupling device for removably attaching the main body to the suction canister.
6. The bypass system in any of items 1 to item 5, wherein the coupling device
   comprises a fastener sized to
   pass through an opening of the suction canister and an opening of the main body.
7. The bypass system in any of items 1 to item 6, wherein the trigger comprises a movable element coupled to an outer wall of the suction canister, the movable element being configured to move from a first position to a second position when a pressure of the suction canister reaches a threshold level.
8. The bypass system in any of items 1 to item 7, further comprising a tactile sensor having a contact surface configured to contact a surface of the movable element in the second position when the main body is coupled to the suction canister.
9. The bypass system in any of items 1 to item 8, wherein the tactile sensor is positioned such that the movable element contacts the tactile sensor when the pressure reaches the threshold level.
10. The bypass system in any of items 1 to item 9, wherein the tactile sensor is coupled to the signal device and configured to activate the signal device when the movable element contacts the tactile sensor.
11. The bypass system in any of items 1 to item 10, further comprising a second suction canister fluidly coupled to the one or more tubes, wherein the trigger is a first trigger and wherein the first trigger is configured to detect pressure within the first suction canister, the device further comprising a second trigger configured to detect pressure within the second suction canister.
12. The bypass system in any of items 1 to item 11, wherein the main body comprises an elongate portion and first and second opposing ends, wherein the opposing ends each comprise a coupling device for coupling the main body to the first and second suction canisters and the elongate body comprises a handle for grasping and carrying the main body and the suction canisters.
13. The bypass system in any of items 1 to item 12, wherein the signal comprises an audible alert.
14. The bypass system in any of items 1 to item 13, wherein the signal comprises a visual alert.
15. The bypass system in any of items 1 to item 14, wherein the signal comprises data and the device further comprises a transmitter for transmitting the data to a remote location.
16. A portable alarm device for use with a negative pressure system, the device comprising:
   a main body configured for removable attachment to a suction cannister, the main body including a handle;
   at least one trigger coupled to the main body and configured to detect a pressure from an interior of the suction cannister; and
   a signal device coupled to the trigger and configured to emit a signal.
17. The device of item 16, wherein the main body comprises a coupling device for removably attaching to the main body to the suction cannister.
18. The device of item 16 or 17, wherein the coupling device comprises a fastener sized to pass through an opening of the suction cannister and an opening of the main body.
19. The device in any of items 16 to item 18, wherein the trigger comprises an expandable element coupled to an outer wall of the suction canister, the expandable element being configured to expand when a pressure of the suction canister reaches a threshold level.
20. The device in any of items 16 to item 19, further comprising a tactile sensor having a contact surface configured to contact a surface of the expandable element of the suction canister when the main body is coupled to the suction cannister.
21. The device in any of items 16 to item 20, wherein the tactile sensor is positioned such that the expandable element contacts the tactile sensor when the pressure reaches the threshold level.
22. The device in any of items 16 to item 21, wherein the tactile sensor is coupled to the signal device and configured to activate the signal device when the expandable element contacts the tactile sensor.
23. The device in any of items 16 to item 23, wherein the main body comprises an elongate body having opposing ends configured for removable attachment to first and second suction cannisters.
24. The device in any of items 16 to item 23, wherein the trigger is a first trigger and wherein the first trigger is configured to detect pressure within the first suction cannister, the device further comprising a second trigger configured to detect pressure within the second suction cannister.
25. The device in any of items 16 to item 24, wherein the elongate body comprises the handle.
26. The device in any of items 16 to item 25, wherein the signal comprises an audible alert.
27. The device in any of items 16 to item 26, wherein the signal comprises a visual alert.
28. The device in any of items 16 to item 27, wherein the signal comprises data and the device further comprises a transmitter for transmitting the data to a remote location.
29. A portable device for use for use with a negative pressure system, the device comprising:
   an elongate body with first and second opposing ends, wherein the first and second ends are configured for removable attachment to first and second suction canisters;
   a first trigger configured to detect a pressure from an interior of the first suction canister and a second trigger configured to detect a pressure from an interior of the second suction canister; and
   a signal device coupled to each trigger and configured to emit a signal
30. The device of item 29, wherein the elongate body comprises a handle.
31. The device of item 29 or 30, wherein the alarm device comprises first and second coupling devices for removably attaching the elongate body to the first and second suction canisters.
32. The device in any of items 29 to item 31, wherein each of the first and second coupling devices comprises a fastener sized to pass through an opening of each of the first and second suction canisters and an opening of the main body.
33. The device in any of items 29 to item 32, wherein the first and second triggers each comprise an expandable element configured to expand when the pressure within one of the first and second suction canisters reaches the threshold level.
34. The device in any of items 29 to item 33, further comprising first and second tactile sensors positioned such that the expandable elements contacts one of the first and second tactile sensors when the pressure reaches the threshold level.
35. The device in any of items 29 to item 34, wherein the first and second tactile sensors are coupled to the signal device and configured to activate the signal device when an expandable element of either one or both of the first and second suction cannisters contacts one of the tactile sensors.

## Claims

1. A portable alarm device for use with a negative pressure system, the device comprising:
a main body configured for removable attachment to a suction cannister, the main body including a handle;
at least one trigger coupled to the main body and configured to detect a pressure from an interior of the suction cannister; and
a signal device coupled to the trigger and configured to emit a signal.

2. The device of claim 1, wherein the main body comprises a coupling device for removably attaching to the main body to the suction cannister.

3. The device of claim 2, wherein the coupling device comprises a fastener sized to pass through an opening of the suction cannister and an opening of the main body.

4. The device of claim 1, wherein the trigger comprises an expandable element coupled to an outer wall of the suction canister, the expandable element being configured to expand when a pressure of the suction canister reaches a threshold level.

5. The device of claim 4, further comprising a tactile sensor having a contact surface configured to contact a surface of the expandable element of the suction canister when the main body is coupled to the suction cannister.

6. The device of claim 5, wherein the tactile sensor is positioned such that the expandable element contacts the tactile sensor when the pressure reaches the threshold level.

7. The device of claim 6, wherein the tactile sensor is coupled to the signal device and configured to activate the signal device when the expandable element contacts the tactile sensor.

8. The device of claim 1, wherein the main body comprises an elongate body having opposing ends configured for removable attachment to first and second suction cannisters.

9. The device of claim 8, wherein the trigger is a first trigger and wherein the first trigger is configured to detect pressure within the first suction cannister, the device further comprising a second trigger configured to detect pressure within the second suction cannister.

10. The device of claim 8, wherein the elongate body comprises the handle.

11. The device of claim 1, wherein the signal comprises an audible alert.

12. The device of claim 1, wherein the signal comprises a visual alert.

13. The device of claim 1, wherein the signal comprises data and the device further comprises a transmitter for transmitting the data to a remote location.

14. A portable device for use for use with a negative pressure system, the device comprising:
an elongate body with first and second opposing ends, wherein the first and second ends are configured for removable attachment to first and second suction canisters;
a first trigger configured to detect a pressure from an interior of the first suction canister and a second trigger configured to detect a pressure from an interior of the second suction canister; and
a signal device coupled to each trigger and configured to emit a signal.

15. The device of claim 14, wherein the alarm device comprises first and second coupling devices for removably attaching the elongate body to the first and second suction canisters.
